# EUROPEAN PATENT SPECIFICATION

(11) **EP 4 340 761 B1**
(45) Date of publication and mention of the grant of the patent: **16.07.2025**
(21) Application number: 22707269.1
(22) Date of filing: 30.01.2022
(51) Int. Cl.: A61B 18/14, A61B 18/00

(54) **SURGICAL INSTRUMENTS METHODS INCORPORATING ULTRASONIC, ELECTROSURGICAL, AND FLUID DELIVERY FUNCTIONALITY**
CHIRURGISCHE INSTRUMENTE MIT ULTRASCHALL-, ELEKTROCHIRURGISCHER UND FLÜSSIGKEITSABGABEFUNKTIONALITÄT
INSTRUMENTS CHIRURGICAUX INCORPORANT UNE FONCTIONNALITÉ ULTRASONORE, ÉLECTROCHIRURGICALE ET DE DISTRIBUTION DE FLUIDE

(30) Priority: 26.02.2021 US 202163154183 P
(43) Date of publication of application: 27.03.2024
(73) Proprietor: Covidien LP, Mansfield, MA 02048 (US)
(72) Inventor: DROCHNER, Thomas E., Boulder, Colorado 80301 (US); COWLEY, Matthew S., Boulder, Colorado 80301 (US); BONN, Kenlyn S., Boulder, Colorado 80301 (US); FAGAN, James R., Boulder, Colorado 80301 (US); LYONS, Michael B., Boulder, Colorado 80301 (US); VAN TOL, David J., Boulder, Colorado 80301 (US)
(74) Representative: Maschio & Soames IP Ltd
(86) International application number: PCT/US2022/014466
(87) International publication number: WO 2022/182476

(56) References cited:
- US-A1- 2014 135 804
- US-A1- 2017 311 974
- US-A1- 2019 201 043
- US-A1- 2019 216 530

## Description

### FIELD

The present disclosure relates to energy-based surgical instruments and, more particularly, to surgical instruments, systems, and methods incorporating ultrasonic, electrosurgical, and fluid delivery functionality to facilitate energy-based tissue treatment.

### BACKGROUND

Ultrasonic surgical instruments and systems utilize ultrasonic energy, i.e., ultrasonic vibrations, to treat tissue. More specifically, ultrasonic surgical instruments and systems utilize mechanical vibration energy transmitted at ultrasonic frequencies to treat tissue. An ultrasonic surgical device may include, for example, an ultrasonic blade and a clamp mechanism to enable clamping of tissue against the blade. Ultrasonic energy transmitted to the blade causes the blade to vibrate at very high frequencies, which allows for heating tissue to treat tissue clamped against or otherwise in contact with the blade.

Electrosurgical instruments and systems conduct Radio Frequency (RF) energy through tissue to treat tissue. An electrosurgical instrument or system may be configured to conduct bipolar RF energy between oppositely charged electrodes and through tissue, e.g., tissue clamped between the electrodes or otherwise in contact therewith, to treat tissue. Alternatively or additionally, an electrosurgical instrument or system may be configured to deliver monopolar RF energy from an active electrode to tissue in contact with the electrode, with the energy returning via a remote return electrode device to complete the circuit.

A fluid, e.g., a conductive fluid such as saline, may be utilized with some electrosurgical instruments and systems, e.g., to couple the RF energy to tissue. The use of fluid may facilitate RF energy-based tissue treatment by controlling temperature, inhibiting sticking, inhibiting smoke production, and/or inhibiting char formation.

An example of an ultrasonic surgical instrument is disclosed in US2019201043 A1.

### SUMMARY

As used herein, the term "distal" refers to the portion that is described which is further from an operator (whether a human surgeon or a surgical robot), while the term "proximal" refers to the portion that is being described which is closer to the operator. Terms including "generally," "about," "substantially," and the like, as utilized herein, are meant to encompass variations, e.g., manufacturing tolerances, material tolerances, use and environmental tolerances, measurement variations, and/or other variations, up to and including plus or minus 10 percent. Further, any or all of the aspects described herein, to the extent consistent, may be used in conjunction with any or all of the other aspects described herein.

The invention provides a surgical system including an ultrasonic transducer, an ultrasonic waveguide coupled to and extending distally from the ultrasonic transducer, and an ultrasonic blade disposed at a distal end of the ultrasonic waveguide. The ultrasonic blade is configured to receive ultrasonic energy produced by the ultrasonic transducer and transmitted along the ultrasonic waveguide to vibrate the ultrasonic blade for treating tissue therewith. The ultrasonic blade is configured to connect to a source of electrosurgical energy for conducting electrosurgical energy to tissue to treat tissue. The ultrasonic blade defines a lumen extending at least partially therethrough to at least one opening. The lumen is configured for fluid communication with a fluid source to enable the delivery of fluid from the fluid source through the lumen and out at least one opening into a surgical site to facilitate electrosurgical tissue treatment.

In an aspect of the present disclosure, the system further includes a housing. Each of the ultrasonic transducer and the fluid source are disposed on or within the housing.

In another aspect of the present disclosure, the fluid is an electrically conductive fluid configured to electrically couple the ultrasonic blade with tissue when the ultrasonic blade is used for electrosurgical tissue treatment.

In another aspect of the present disclosure, vibrating the ultrasonic blade heats the ultrasonic blade, and the delivery of fluid from the fluid source through the lumen and out the at least one opening into a surgical site cools the ultrasonic blade.

In still another aspect of the present disclosure, the system further includes a jaw member movable relative to the ultrasonic blade from a spaced-apart position to an approximated position for clamping tissue therebetween.

In yet another aspect of the present disclosure, the jaw member includes a structural body and a jaw liner disposed within the structural body. The jaw liner defines a tissue contacting surface positioned to oppose the ultrasonic blade in the approximated position.

In still yet another aspect of the present disclosure, the structural body is configured to connect to the source of electrosurgical energy such that the structural body and ultrasonic blade are configured to conduct bipolar electrosurgical energy through tissue disposed therebetween to treat tissue. Alternatively, the structural body includes at least one electrically conductive surface disposed thereon that is configured to connect to the source of electrosurgical energy such that the at least one electrically conductive surface and ultrasonic blade are configured to conduct bipolar electrosurgical energy through tissue disposed therebetween to treat tissue.

In another aspect of the present disclosure, the ultrasonic blade is configured to conduct monopolar electrosurgical energy to tissue to treat tissue.

The invention also provides another system, including an ultrasonic transducer, an ultrasonic waveguide coupled to and extending distally from the ultrasonic transducer, and an ultrasonic blade disposed at a distal end of the ultrasonic waveguide. The ultrasonic blade is configured to receive ultrasonic energy produced by the ultrasonic transducer and transmitted along the ultrasonic waveguide to vibrate the ultrasonic blade for treating tissue therewith. The ultrasonic blade is configured to connect to a source of electrosurgical energy for conducting electrosurgical energy to tissue to treat tissue. A jaw member is movable relative to the ultrasonic blade from a spaced-apart position to an approximated position for clamping tissue therebetween. The jaw member defines a lumen configured for fluid communication with a fluid source to enable the delivery of fluid from the fluid source, through the lumen, out at least one opening defined in the jaw member, and into a surgical site to facilitate electrosurgical tissue treatment.

In an aspect of the present disclosure, the system further includes a housing. Each of the ultrasonic transducer and the fluid source are disposed on or within the housing.

In another aspect of the present disclosure, the fluid is an electrically conductive fluid configured to electrically couple the ultrasonic blade with tissue when the ultrasonic blade is used for electrosurgical tissue treatment.

In another aspect of the present disclosure, vibrating the ultrasonic blade heats the ultrasonic blade, and the delivery of fluid from the fluid source through the lumen and out the at least one opening is directed towards the blade to cool the ultrasonic blade.

In yet another aspect of the present disclosure, the jaw member includes a structural body and a jaw liner disposed within the structural body. The jaw liner defines a tissue contacting surface positioned to oppose the ultrasonic blade in the approximated position.

In still another aspect of the present disclosure, the structural body is configured to connect to the source of electrosurgical energy such that the structural body and ultrasonic blade are configured to conduct bipolar electrosurgical energy through tissue disposed therebetween to treat tissue. Alternatively, the structural body includes at least one electrically conductive surface disposed thereon that is configured to connect to the source of electrosurgical energy such that the at least one electrically conductive surface and ultrasonic blade are configured to conduct bipolar electrosurgical energy through tissue disposed therebetween to treat tissue.

In still yet another aspect of the present disclosure, the ultrasonic blade is configured to conduct monopolar electrosurgical energy to tissue to treat tissue.

A method, not forming part of the invention, of surgery in accordance with the present disclosure includes transmitting ultrasonic energy to an ultrasonic blade to vibrate the ultrasonic blade for treating tissue therewith, conducting electrosurgical energy from the ultrasonic blade to tissue for treating tissue therewith, and supplying fluid from the ultrasonic blade to tissue.

In aspects, the transmitting is performed in conjunction with the supplying such that the supplying of the fluid facilitates electrosurgical tissue treatment. Additionally or alternatively, the supplying is performed after the transmitting such that the supplying of the fluid serves to cool the ultrasonic blade.

### BRIEF DESCRIPTION OF THE DRAWINGS

The above and other aspects and features of the present disclosure will become more apparent in light of the following detailed description when taken in conjunction with the accompanying drawings wherein like reference numerals identify similar or identical elements.
FIG. 1 illustrates a surgical system provided in accordance with the present disclosure including a surgical instrument, a surgical generator and, in some aspects, a return electrode device;
FIG. 2 is a schematic illustration of a robotic surgical system provided in accordance with the present disclosure;
FIG. 3 is an enlarged, longitudinal, cross-sectional view of a distal portion of an end effector assembly configured for use with the surgical instrument of FIG. 1, the robotic surgical system of FIG. 2, or any other suitable surgical instrument or system;
FIG. 4 is an enlarged, longitudinal, cross-sectional view of a distal portion of another end effector assembly configured for use with the surgical instrument of FIG. 1, the robotic surgical system of FIG. 2, or any other suitable surgical instrument or system;
FIG. 5 is an enlarged, transverse, cross-sectional view of the distal portion of the end effector assembly of FIG. 3; and
FIG. 6 is an enlarged, transverse, cross-sectional view of a distal portion of another end effector assembly configured for use with the surgical instrument of FIG. 1, the robotic surgical system of FIG. 2, or any other suitable surgical instrument or system.

### DETAILED DESCRIPTION

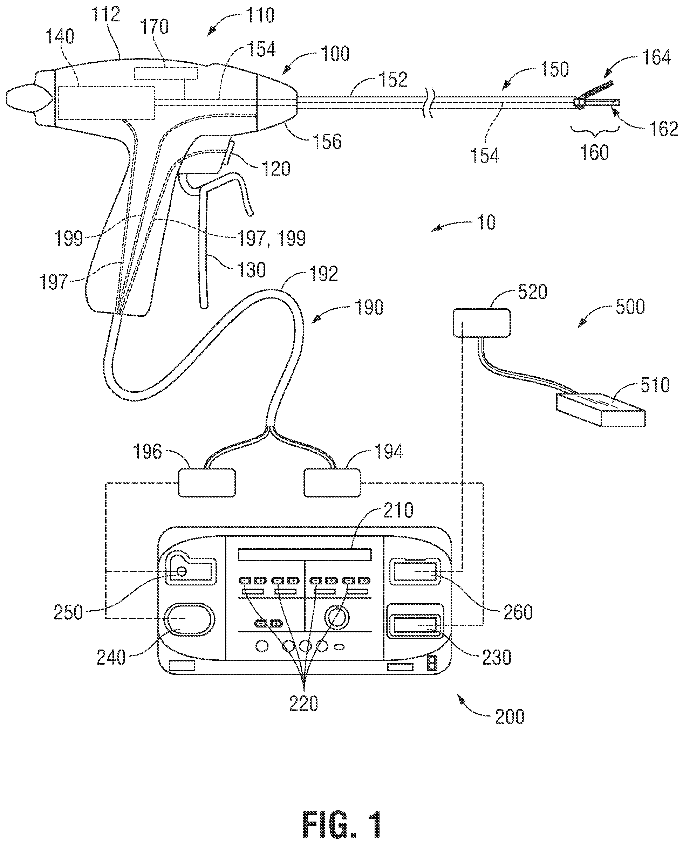

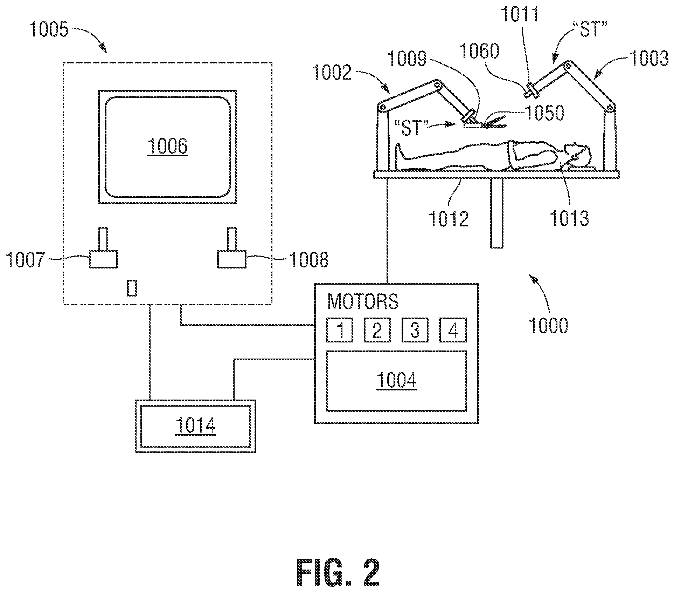

Referring to FIG. 1, a surgical system provided in accordance with aspects of the present disclosure is shown generally identified by reference numeral 10 including a surgical instrument 100, a surgical generator 200, and, in some aspects, a return electrode device 500, e.g., including a return pad 510. Surgical instrument 100 includes a handle assembly 110, an elongated assembly 150 extending distally from handle assembly 110, an end effector assembly 160 disposed at a distal end of elongated assembly 150, and a cable assembly 190 operably coupled with handle assembly 110 and extending therefrom for connection to surgical generator 200. As an alternative to handle assembly 110, surgical instrument 100 may include a robotic attachment housing for releasable engagement with a robotic arm of a robotic surgical system such as, for example, robotic surgical system 1000 (FIG. 2) detailed below.

Surgical generator 200 includes a display 210, a plurality user interface features 220, e.g., buttons, touch screens, switches, etc., an ultrasonic plug port 230, a bipolar electrosurgical plug port 240, and active and return monopolar electrosurgical plug ports 250, 260, respectively. Surgical generator 200 is configured to produce ultrasonic drive signals for output through ultrasonic plug port 230 to surgical instrument 100 to activate surgical instrument 100 in an ultrasonic mode and to provide electrosurgical energy, e.g., RF bipolar energy, for output through bipolar electrosurgical plug port 240 and/or RF monopolar energy for output through active monopolar electrosurgical port 250 to surgical instrument 100 to activate surgical instrument 100 in one or more electrosurgical modes. It is also contemplated that one or more common ports (not shown) may be configured to act as any two or more of ports 230-260. In monopolar configurations, plug 520 of return electrode device 500 is configured to connect to return monopolar electrosurgical plug port 260.

Continuing with reference to FIG. 1, handle assembly 110 includes a housing 112 defining a body portion and a fixed handle portion. Handle assembly 110 further includes an activation button 120 and a clamp trigger 130. The body portion of housing 112 is configured to support an ultrasonic transducer 140. Ultrasonic transducer 140 may be permanently engaged with the body portion of housing 112 or removable therefrom. Ultrasonic transducer 140 includes a piezoelectric stack or other suitable ultrasonic transducer components electrically coupled to surgical generator 200, e.g., via one or more of first electrical lead wires 197, to enable communication of ultrasonic drive signals to ultrasonic transducer 140 to drive ultrasonic transducer 140 to produce ultrasonic vibration energy that is transmitted along a waveguide 154 of elongated assembly 150 to blade 162 of end effector assembly 160 of elongated assembly 150, as detailed below. An activation button 120 is disposed on housing 112 and coupled to or between ultrasonic transducer 140 and/or surgical generator 200, e.g., via one or more of first electrical lead wires 197, to enable activation of ultrasonic transducer 140 in response to depression of activation button 120. In some configurations, activation button 120 may include an ON/OFF switch. In other configurations, activation button 120 may include multiple actuation switches to enable activation from an OFF position to different actuated positions corresponding to different activation settings, e.g., a first actuated position corresponding to a first activation setting and a second actuated position corresponding to a second activation setting. In still other configurations, separate activation buttons may be provided, e.g., a first actuation button for activating a first activation setting and a second activation button for activating a second activation setting.

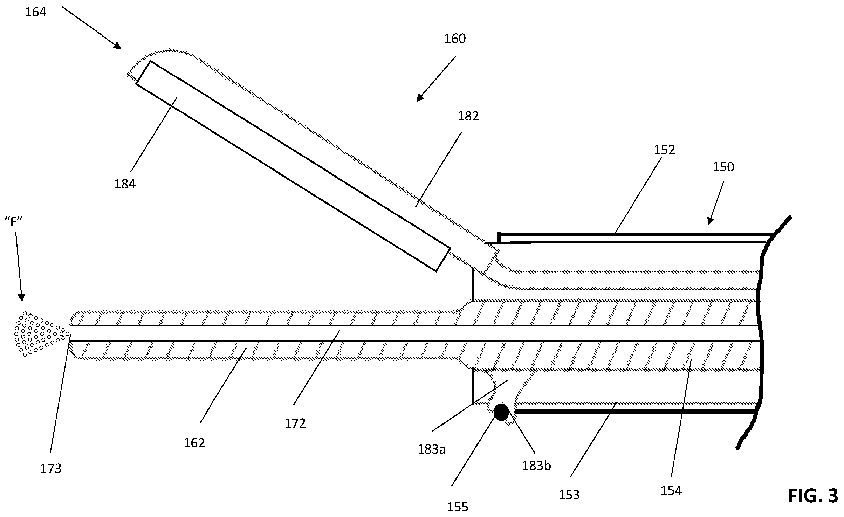

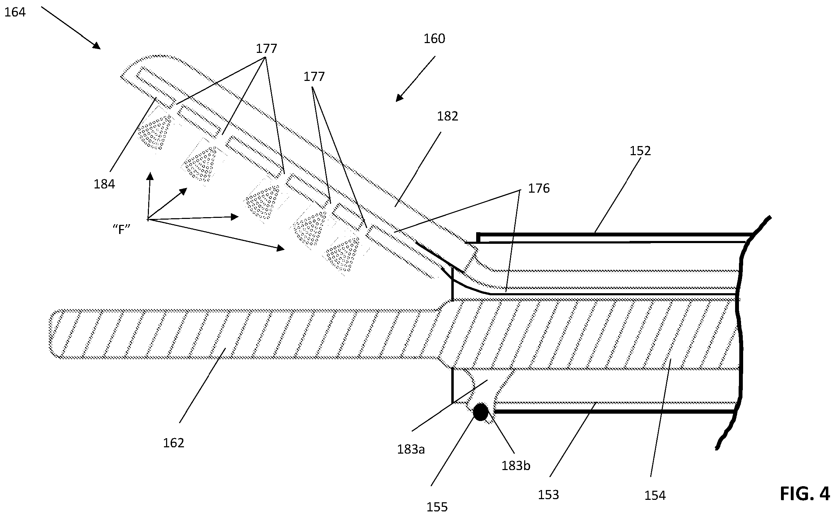

A fluid source 170 is disposed within housing 112 in fluid communication with a lumen 172 (FIG. 3) extending through at least a portion of waveguide 154 and/or blade 162 (and/or a lumen 176 (FIG. 4) extending through jaw member 164). The fluid source 170 may include a fluid reservoir and/or a pump and may be configured to store and/or pump any suitable fluid "F" (FIG. 3) including conductive fluid, e.g., saline, through the lumen 172 (FIG. 3) (and/or lumen 176 (FIG. 4)). In configurations, fluid source 170 is external to housing 112 (mounted thereon or separate therefrom and connected via suitable tubing).

Elongated assembly 150 of surgical instrument 100 includes an outer drive sleeve 152, an inner support sleeve 153 (FIG. 3) disposed within outer drive sleeve 152, a waveguide 154 extending through inner support sleeve 153 (FIG. 3), a drive assembly (not shown), a rotation knob 156, and an end effector assembly 160 including a blade 162 and a jaw member 164. Rotation knob 156 is rotatable in either direction to rotate elongated assembly 150 in either direction relative to handle assembly 110. The drive assembly operably couples a proximal portion of outer drive sleeve 152 to clamp trigger 130 of handle assembly 110. A distal portion of outer drive sleeve 152 is operably coupled to jaw member 164 and a distal end of inner support sleeve 153 (FIG. 3) pivotably supports jaw member 164. As such, clamp trigger 130 is selectively actuatable to thereby move outer drive sleeve 152 about inner support sleeve 153 (FIG. 3) to pivot jaw member 164 relative to blade 162 of end effector assembly 160 from a spaced apart position to an approximated position for clamping tissue between jaw member 164 and blade 162. The configuration of outer and inner sleeves 152, 153 (FIG. 3) may be reversed, e.g., wherein outer sleeve 152 is the support sleeve and inner sleeve 153 (FIG. 3) is the drive sleeve. Other suitable drive structures as opposed to a sleeve are also contemplated such as, for example, drive rods, drive cables, drive screws, etc.

Referring still to FIG. 1, the drive assembly may be tuned to provide a jaw clamping force, or jaw clamping force within a jaw clamping force range, to tissue clamped between jaw member 164 and blade 162 or may include a force limiting feature whereby the clamping force applied to tissue clamped between jaw member 164 and blade 162 is limited to a particular jaw clamping force or a jaw clamping force within a jaw clamping force range.

Waveguide 154, as noted above, extends from handle assembly 110 through the inner support sleeve. Waveguide 154 includes blade 162 disposed at a distal end thereof. Blade 162 may be integrally formed with waveguide 154, separately formed and subsequently attached (permanently or removably) to waveguide 154, or otherwise operably coupled with waveguide 154. Waveguide 154 and/or blade 162 may be formed from titanium, a titanium alloy, or other suitable electrically conductive material(s), although non-conductive materials are also contemplated. Waveguide 154 includes a proximal connector (not shown), e.g., a threaded male connector, configured for engagement, e.g., threaded engagement within a threaded female receiver, of ultrasonic transducer 140 such that ultrasonic motion produced by ultrasonic transducer 140 is transmitted along waveguide 154 to blade 162 for treating tissue clamped between blade 162 and jaw member 164 or positioned adjacent to blade 162.

Cable assembly 190 of surgical instrument 100 includes a cable 192, an ultrasonic plug 194, and an electrosurgical plug 196. Ultrasonic plug 194 is configured for connection with ultrasonic plug port 230 of surgical generator 200 while electrosurgical plug 196 is configured for connection with bipolar electrosurgical plug port 240 of surgical generator 200 and/or active monopolar electrosurgical plug port 250 of surgical generator 200. In configurations where generator 200 includes a common port, cable assembly 190 may include a common plug (not shown) configured to act as both the ultrasonic plug 194 and the electrosurgical plug 196. Plural first electrical lead wires 197 electrically coupled to ultrasonic plug 194 extend through cable 192 and into handle assembly 110 for electrical connection to ultrasonic transducer 140 and/or activation button 120 to enable the selective supply of ultrasonic drive signals from surgical generator 200 to ultrasonic transducer 140 upon activation of activation button 120 in an ultrasonic mode. In addition, plural second electrical lead wires 199 are electrically coupled to electrosurgical plug 196 and extend through cable 192 into handle assembly 110. In bipolar configurations, separate second electrical lead wires 199 are electrically coupled to waveguide 154 and jaw member 164 (and/or different portions of jaw member 164) such that, as detailed below, bipolar electrosurgical energy may be conducted between blade 162 and jaw member 164 (and/or between different portions of jaw member 164). In monopolar configurations, an electrical lead wire 199 is electrically coupled to waveguide 154 such that, as also detailed below, monopolar electrosurgical energy may be supplied to tissue from blade 162. Alternatively, an electrical lead wire 199 may electrically couple to jaw member 164 in the monopolar configuration to enable monopolar electrosurgical energy to be supplied to tissue from jaw member 164. One or more second electrical lead wires 199 is electrically coupled to activation button 120 to enable the selective supply of electrosurgical energy from surgical generator 200 to waveguide 154 and/or jaw member 164 upon activation of activation button 120 in an electrosurgical mode.

As an alternative to a remote generator 200, surgical system 10 may be at least partially cordless in that it incorporates an ultrasonic generator, an electrosurgical generator, and/or a power source, e.g., a battery, thereon or therein. In this manner, the connections from surgical instrument 100 to external devices, e.g., generator(s) and/or power source(s), is reduced or eliminated.

With reference to FIG. 2, a robotic surgical system in accordance with the aspects and features of the present disclosure is shown generally identified by reference numeral 1000. For the purposes herein, robotic surgical system 1000 is generally described. Aspects and features of robotic surgical system 1000 not germane to the understanding of the present disclosure are omitted to avoid obscuring the aspects and features of the present disclosure in unnecessary detail.

Robotic surgical system 1000 generally includes a plurality of robot arms 1002, 1003; a control device 1004; and an operating console 1005 coupled with control device 1004. Operating console 1005 may include a display device 1006, which may be set up in particular to display three dimensional images; and manual input devices 1007, 1008, by means of which a person (not shown), for example a surgeon, may be able to telemanipulate robot arms 1002, 1003 in a first operating mode. Robotic surgical system 1000 may be configured for use on a patient 1013 lying on a patient table 1012 to be treated in a minimally invasive manner. Robotic surgical system 1000 may further include a database 1014, in particular coupled to control device 1004, in which are stored, for example, pre-operative data from patient 1013 and/or anatomical atlases.

Each of the robot arms 1002, 1003 may include a plurality of members, which are connected through joints, and an attaching device 1009, 1011, to which may be attached, for example, a surgical tool "ST" supporting an end effector 1050, 1060. One of the surgical tools "ST" may be ultrasonic surgical instrument 100 (FIG. 1), e.g., configured for use in both an ultrasonic mode and an electrosurgical (bipolar and/or monopolar) mode, wherein manual actuation features, e.g., actuation button 120 (FIG. 1), clamp lever 130 (FIG. 1), etc., are replaced with robotic inputs. In such configurations, robotic surgical system 1000 may include or be configured to connect to an ultrasonic generator, an electrosurgical generator, and/or a power source. The other surgical tool "ST" may include any other suitable surgical instrument, e.g., an endoscopic camera, other surgical tool, etc. Robot arms 1002, 1003 may be driven by electric drives, e.g., motors, that are connected to control device 1004. Control device 1004 (e.g., a computer) may be configured to activate the motors, in particular by means of a computer program, in such a way that robot arms 1002, 1003, their attaching devices 1009, 1011, and, thus, the surgical tools "ST" execute a desired movement and/or function according to a corresponding input from manual input devices 1007, 1008, respectively. Control device 1004 may also be configured in such a way that it regulates the movement of robot arms 1002, 1003 and/or of the motors.

Referring to FIG. 3, end effector assembly 160 of surgical instrument 100 of surgical system 10 (FIG. 1) is detailed, although end effector assembly 160 may be utilized with any other suitable surgical instrument and/or surgical system. End effector assembly 160 includes a blade 162 and a jaw member 164. Blade 162 may define a linear configuration, may define a curved configuration, or may define any other suitable configuration, e.g., straight and/or curved surfaces, portions, and/or sections; one or more convex and/or concave surfaces, portions, and/or sections; etc. With respect to curved configurations, blade 162, more specifically, may be curved in any direction relative to jaw member 164, for example, such that the distal tip of blade 162 is curved towards jaw member 164, away from jaw member 164, or laterally (in either direction) relative to jaw member 164. Further, blade 162 may be formed to include multiple curves in similar directions, multiple curves in different directions within a single plane, and/or multiple curves in different directions in different planes. In addition, blade 162 may additionally or alternatively be formed to include any suitable features, e.g., a tapered configuration, various different cross-sectional configurations along its length, cut outs, indents, edges, protrusions, straight surfaces, curved surfaces, angled surfaces, wide edges, narrow edges, and/or other features.

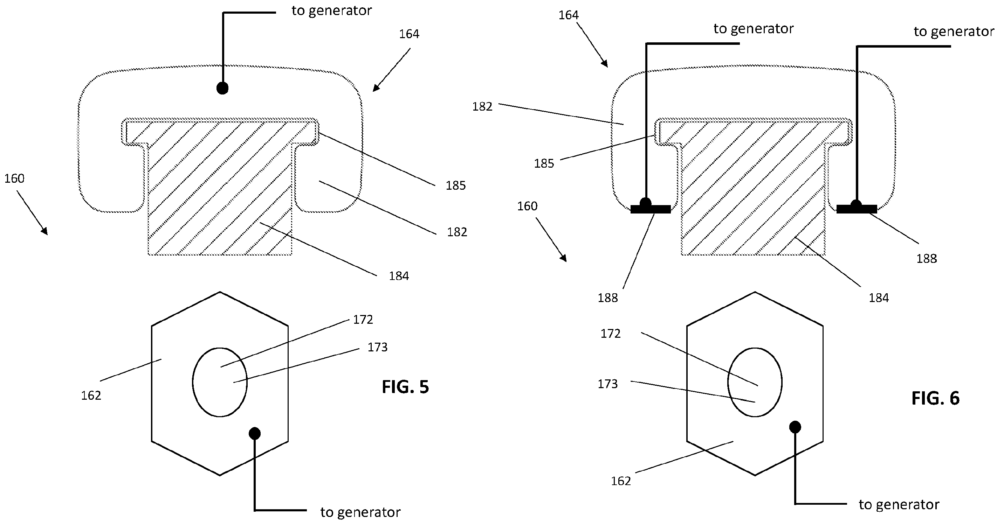

Blade 162 may define a polygonal, rounded polygonal, or any other suitable cross-sectional configuration(s) (see FIG. 5). Waveguide 154 or at least the portion of waveguide 154 proximally adjacent blade 162, may define a cylindrical shaped configuration. Plural tapered surfaces (not shown) may interconnect the cylindrically shaped waveguide 154 with the polygonal (rounded edge polygonal, or other suitable shape) configuration of blade 162 to define smooth transitions between the body of waveguide 154 and blade 162.

Blade 162 may be wholly or selectively coated with a suitable material, e.g., a non-stick material, an electrically insulative material, an electrically conductive material, combinations thereof, etc. Suitable coatings and/or methods of applying coatings include but are not limited to Teflon^{®}, polyphenylene oxide (PPO), deposited liquid ceramic insulative coatings; thermally sprayed coatings, e.g., thermally sprayed ceramic; Plasma Electrolytic Oxidation (PEO) coatings; anodization coatings; sputtered coatings, e.g., silica; ElectroBond^{®} coating available from Surface Solutions Group of Chicago, IL, USA; or other suitable coatings and/or methods of applying coatings.

Waveguide 154 and/or blade 162 defines lumen 172 extending at least partially therethrough. Lumen 172, as noted above, is disposed in fluid communication with fluid source 170 (FIG. 1) to enable the delivery, e.g., pumping, of fluid "F" through lumen 172 and into a surgical site via one or more openings 173 in fluid communication with lumen 172. Although one opening 173 is shown on a distal face of blade 162, any other suitable number and/or positioning of openings 173 may be provided to facilitate introduction of fluid "F" into the surgical site such as, for example, openings 173 along at least a portion of a length of either or both sides of blade 162, openings 173 along at least a portion of a length of a top (jaw member facing side) of blade 162, and/or openings 173 along at least a portion of a length of a bottom (opposite the jaw member facing side) of blade 162. Further, the one or more openings 173 may be configured to deliver fluid "F" in any suitable manner, e.g., spray, jet, drip, etc., and/or direction(s).

With additional reference to FIG. 5, blade 162, as noted above, in addition to receiving ultrasonic energy transmitted along waveguide 154 from ultrasonic transducer 140 (FIG. 1), is adapted to connect to generator 200 (FIG. 1) to enable the supply of RF energy to blade 162 for conduction to tissue in contact therewith or coupled thereto (e.g., via conductive fluid "F," for example). In bipolar configurations, RF energy is conducted between blade 162 and jaw member 164 (or between portions of jaw member 164 and/or blade 162) and through tissue (and/or fluid "F") disposed therebetween to treat tissue. In monopolar configurations, RF energy is conducted from blade 162, serving as the active electrode, to tissue in contact therewith or coupled thereto (e.g., via fluid "F") and is ultimately returned to generator 200 (FIG. 1) via return device 500 (FIG. 1), serving as the passive or return electrode.

Continuing with reference to FIG. 3, jaw member 164 of end effector assembly 160 includes a more rigid structural body 182 and a more compliant jaw liner 184. Structural body 182 may be formed from an electrically conductive material, e.g., stainless steel, and/or may include electrically conductive portions. Structural body 182 includes a pair of proximal flanges 183a that are pivotably coupled to the inner support sleeve 153 via receipt of pivot bosses (not shown) of proximal flanges 183a within corresponding openings (not shown) defined within the inner support sleeve 153 and operably coupled with outer drive sleeve 152 via a drive pin 155 secured relative to outer drive sleeve 152 and pivotably received within apertures 183b defined within proximal flanges 183a. As such, sliding of outer drive sleeve 152 about inner support sleeve 153 pivots jaw member 164 relative to blade 162 from a spaced apart position to an approximated position to clamp tissue between jaw liner 184 of jaw member 164 and blade 162.

Referring also to FIG. 5, structural body 182 may be adapted to connect to a source of electrosurgical energy, e.g., generator 200 (FIG. 1), and, in a bipolar electrosurgical mode, is charged to a different potential as compared to blade 162 to enable the conduction of bipolar electrosurgical (e.g., RF) energy through tissue clamped therebetween (and/or through fluid "F"), to treat the tissue. In a monopolar electrosurgical mode, structural body 182 may be un-energized, may be charged to the same potential as compared to blade 162 (thus both defining the active electrode), or may be energized while blade 162 is not energized (wherein structural body 182 defines the active electrode). In either monopolar configuration, energy is returned to generator 200 (FIG. 1) via return device 500 (FIG. 1), which serves as the passive or return electrode.

With momentary reference to FIG. 6, as an alternative to the entirety of structural body 182 of jaw member 164 being connected to generator 200 (FIG. 1), the structural body may be formed from or embedded at least partially in an insulative material, e.g., an overmolded plastic. In such configurations, electrically conductive surfaces 188, e.g., in the form of plates, may be disposed on or captured by the overmolded plastic to define electrodes on either side of jaw liner 184 on the blade facing side of jaw member 164. The electrically conductive surfaces 188, in such aspects, are connected to generator 200 (FIG. 1) and may be energized for use in bipolar and/or monopolar configurations, e.g., energized to the same potential as one another and/or blade 162 and/or different potentials as one another and/or blade 162. In aspects, electrically conductive surfaces 188 are disposed at additional or alternative locations on jaw member 164, e.g., along either or both sides thereof, along a back surface thereof, etc.

Returning to FIGS. 3 and 5, jaw liner 184 is shaped complementary to a cavity 185 (FIG. 5) defined within structural body 182, e.g., defining a T-shaped configuration, to facilitate receipt and retention therein, although other configurations are also contemplated. Jaw liner 184 is fabricated from an electrically insulative, compliant material such as, for example, polytetrafluoroethylene (PTFE). The compliance of jaw liner 184 enables blade 162 to vibrate while in contact with jaw liner 184 without damaging components of ultrasonic surgical instrument 100 (FIG. 1) and without compromising the hold on tissue clamped between jaw member 164 and blade 162. The insulation of jaw liner 184 maintains electrical isolation between blade 162 and structural body 182 of jaw member 164, thereby inhibiting shorting.

Turning to FIG. 4, in some aspects, rather than or in addition to blade 162 defining a lumen 172 (FIG. 3) for delivery of fluid "F" to the surgical site, a lumen 176 disposed in fluid communication with fluid source 170 (FIG. 1) may extend through elongated assembly 150 and at least partially through jaw member 164 to enable the delivery, e.g., pumping, of fluid "F" through lumen 176 and into a surgical site via one or more openings 177 in fluid communication with lumen 176. A plurality of openings 177 are shown defined through jaw liner 184 and spaced apart along the length thereof such that fluid "F" is directed towards blade 162; however, any other suitable number and/or positioning of openings 177 may be provided to facilitate introduction of fluid "F" from jaw member 164 into the surgical site. Further, the one or more openings 177 may be configured to deliver fluid "F" in any suitable manner(s), e.g., spray, jet, drip, etc., and/or direction(s).

With general reference to FIGS. 1, 3, and 5, as noted above, end effector assembly 160 is configured for use in an ultrasonic mode and/or one or more electrosurgical modes; the modes may operate consecutively, overlapping, alternatingly, simultaneous, and/or in any other suitable manner. Further, end effector assembly 160 may be configured to supply fluid "F" to tissue, before, after, and/or together with energy delivery (in either or both modes). Various non-limiting use modes of end effector assembly 160 are detailed below.

With respect to the ultrasonic mode, upon activation, an ultrasonic drive signal is provided from surgical generator 200 to ultrasonic transducer 140 to generate ultrasonic energy that is transmitted from ultrasonic transducer 140 along waveguide 154 to blade 162 to thereby vibrate blade 162 for treating tissue in contact with or adjacent to blade 162. More specifically, in the ultrasonic mode: ultrasonic energy may be supplied to blade 162 to treat, e.g., seal and/or transect, tissue clamped between blade 162 and jaw liner 184 of jaw member 164; ultrasonic energy may be supplied to blade 162 to treat, e.g., transect, perform an otomy, backscore, etc., tissue in contact with or adjacent to blade 162 (with jaw member 164 disposed in the spaced apart or approximated position), statically or dynamically; and/or ultrasonic energy may be supplied to blade 162 to treat, e.g., plunge, spot coagulate, etc., tissue utilizing the distal end of blade 162. The ultrasonic mode may include one or more energy level settings such as, for example, a first, e.g., LOW, setting and a second, e.g., HIGH, setting. The first and second energy level settings may correspond to different vibration velocities of blade 162.

The one or more electrosurgical energy modes may include bipolar electrosurgical modes and/or monopolar electrosurgical modes. With respect to bipolar electrosurgical tissue treatment, bipolar electrosurgical energy is conducted between blade 162 and structural body 182 of jaw member 164 (or surfaces 188 of jaw member (FIG. 6)) to treat, e.g., seal, tissue clamped between blade 162 and jaw liner 184. Bipolar electrosurgical tissue treatment may be utilized simultaneously or otherwise in cooperation with the ultrasonic mode, e.g., in the first energy level setting, to facilitate treating, e.g., sealing, tissue. Other suitable configurations for bipolar electrosurgical tissue treatment are also contemplated.

Monopolar electrosurgical tissue treatment involves the supply of electrosurgical energy from blade 162 (with jaw member 164 un-energized), from jaw member 164 (with blade 162 un-energized), or from both blade 162 and jaw member 164 (with both energized to the same potential) to tissue to treat, e.g., transect and/or spot coagulate, tissue. Monopolar electrosurgical tissue treatment utilizes a remote return electrode device, e.g., return pad 510 of device 500 (see FIG. 1) attached to the patient's skin, to safely return energy to generator 200.

Delivery of fluid "F," e.g., conductive fluid such as saline, together with the supply of electrosurgical energy may be used to facilitate tissue treatment, in the bipolar and/or monopolar modes. For example, the use of both electrosurgical energy (bipolar or monopolar), and conductive fluid "F" enables the delivery of energy to tissue while regulating the tissue temperature, e.g., at or below about 100°C. Unlike conventional electrosurgical devices which typically operate at high temperatures, the use of conductive fluid "F" may be utilized to decrease temperature and/or to reduce or eliminate sticking, smoke production, and/or char formation. Additionally or alternatively, the use of a conductive fluid "F" may facilitate coupling the electrode(s) to tissue, enabling tissue treatment without direct contact between the electrode(s) and tissue, thereby expanding the potential treatment area. In aspects, fluid "F" may also be delivered during an ultrasonic mode of operation.

Fluid delivery in conjunction with electrosurgical energy supply may be performed, for example, as detailed in US Patent Application Publication No. 2001/0032002, titled "FLUID DELIVERY SYSTEM AND CONTROLLER FOR ELECTROSURGICAL DEVICES" and filed on March 1, 2001. Other fluid enhanced electrosurgical (and/or ultrasonic) energy implementations are also contemplated.

In configurations where the fluid "F" is delivered through lumen 172 of waveguide 154 and blade 162, this flow of fluid "F" through blade 162 also serves to cool blade 162. As such, after activation in an ultrasonic mode, for example, fluid "F" may be delivered to a surgical site in preparation for or together with the supply of electrosurgical energy to facilitate electrosurgical tissue treatment, while providing the additional benefit of cooling blade 162. Likewise, where the fluid "F" is delivered from jaw member 164, the supply of fluid onto blade 162 may also facilitate cooling of blade 162 after an ultrasonic activation.

The use of non-conductive fluid "F," while not providing the electrical coupling features of conductive fluid "F," may still provide certain advantages over dry bipolar and/or monopolar electrosurgical modes including, for example, reduced occurrence of tissue sticking, charring, and/or smoke production. Further, a non-conductive fluid "F" may also serve to cool blade 162 after an ultrasonic activation, similarly as detailed above. Other suitable fluids are also contemplated such as irrigating fluids for irrigating the surgical site, fluids for cleaning tissue, medicaments to treat tissue, contrast agents, adhesives, etc. These fluids may be utilized for a surgical purpose while, in aspects, also facilitating cooling of blade 162. Further, as an alternative to pumping fluid, fluid source 170 (FIG. 1) may be configured to suction tissue through lumen 172, e.g., for aspiration, removing fluid form the surgical site, etc. Such suction of fluid may likewise facilitate cooling of blade 162 as the fluid is drawn into and through blade 162.

While several aspects of the disclosure have been detailed above and are shown in the drawings, it is not intended that the disclosure be limited thereto, as it is intended that the disclosure be as broad in scope as the art will allow and that the specification be read likewise. Therefore, the above description and accompanying drawings should not be construed as limiting, but merely as exemplifications of particular aspects. The scope of the invention is defined by the appended claims.

## Claims

1. A surgical system (10), comprising:
an ultrasonic transducer (140);
an ultrasonic waveguide (154) coupled to and extending distally from the ultrasonic transducer; and
an ultrasonic blade (162) disposed at a distal end of the ultrasonic waveguide and configured to receive ultrasonic energy produced by the ultrasonic transducer and transmitted along the ultrasonic waveguide to vibrate the ultrasonic blade for treating tissue therewith, the ultrasonic blade configured to connect to a source of electrosurgical energy (200) for conducting electrosurgical energy to tissue to treat tissue, **characterized in that** the ultrasonic blade defines a lumen (172) extending at least partially therethrough to at least one opening (173), the lumen configured for fluid communication with a fluid source (170) to enable the delivery of fluid from the fluid source through the lumen and out the at least one opening into a surgical site to facilitate electrosurgical tissue treatment.

2. The surgical system according to claim 1, further comprising a housing (112), wherein each of the ultrasonic transducer and the fluid source are disposed on or within the housing.

3. The surgical system according to claim 1, further comprising the fluid source, wherein the fluid is an electrically conductive fluid configured to electrically couple the ultrasonic blade with tissue when the ultrasonic blade is used for electrosurgical tissue treatment.

4. The surgical system according to claim 1, wherein vibrating the ultrasonic blade heats the ultrasonic blade, and wherein the delivery of fluid from the fluid source through the lumen and out the at least one opening into a surgical site cools the ultrasonic blade.

5. The surgical system according to claim 1, further comprising a jaw member (164) movable relative to the ultrasonic blade from a spaced-apart position to an approximated position for clamping tissue therebetween.

6. The surgical system according to claim 5, wherein the jaw member includes a structural body (182) and a jaw liner (184) disposed within the structural body, the jaw liner defining a tissue contacting surface positioned to oppose the ultrasonic blade in the approximated position.

7. The surgical instrument according to claim 6, wherein the structural body is configured to connect to the source of electrosurgical energy and wherein the structural body and the ultrasonic blade are configured to conduct bipolar electrosurgical energy through tissue disposed therebetween to treat tissue.

8. The surgical system according to claim 6, wherein the structural body includes at least one electrically conductive surface (188) disposed thereon that is configured to connect to the source of electrosurgical energy, and wherein the at least one electrically conductive surface and ultrasonic blade are configured to conduct bipolar electrosurgical energy through tissue disposed therebetween to treat tissue.

9. The surgical system according to claim 1, wherein the ultrasonic blade is configured to conduct monopolar electrosurgical energy to tissue to treat tissue.

10. A surgical system (10), comprising:
an ultrasonic transducer (140);
an ultrasonic waveguide (154) coupled to and extending distally from the ultrasonic transducer;
an ultrasonic blade (162) disposed at a distal end of the ultrasonic waveguide and configured to receive ultrasonic energy produced by the ultrasonic transducer and transmitted along the ultrasonic waveguide to vibrate the ultrasonic blade for treating tissue therewith, the ultrasonic blade configured to connect to a source of electrosurgical energy (200) for conducting electrosurgical energy to tissue to treat tissue; and
a jaw member (182) movable relative to the ultrasonic blade from a spaced-apart position to an approximated position for clamping tissue therebetween,
**characterized in that**
the jaw member defines a lumen (176) configured for fluid communication with a fluid source (170) to enable the delivery of fluid from the fluid source, through the lumen, out at least one opening (177) defined in the jaw member, and into a surgical site to facilitate electrosurgical tissue treatment.

11. The surgical system according to claim 10, further comprising a housing (112), wherein each of the ultrasonic transducer and the fluid source are disposed on or within the housing.

12. The surgical system according to claim 10, further comprising the fluid source, wherein the fluid is an electrically conductive fluid configured to electrically couple the ultrasonic blade with tissue when the ultrasonic blade is used for electrosurgical tissue treatment.

13. The surgical system according to claim 10, wherein vibrating the ultrasonic blade heats the ultrasonic blade, and wherein the delivery of fluid from the fluid source through the lumen and out the at least one opening is directed towards the ultrasonic blade to cool the ultrasonic blade.

14. The surgical system according to claim 10, wherein the jaw member includes a structural body (182) and a jaw liner (184) disposed within the structural body, the jaw liner defining a tissue contacting surface positioned to oppose the ultrasonic blade in the approximated position, optionally wherein the structural body is configured to connect to the source of electrosurgical energy and wherein the structural body and ultrasonic blade are configured to conduct bipolar electrosurgical energy through tissue disposed therebetween to treat tissue, or
wherein the structural body includes at least one electrically conductive surface (188) disposed thereon that is configured to connect to the source of electrosurgical energy, and wherein the at least one electrically conductive surface and ultrasonic blade are configured to conduct bipolar electrosurgical energy through tissue disposed therebetween to treat tissue.

15. The surgical system according to claim 10, wherein the ultrasonic blade is configured to conduct monopolar electrosurgical energy to tissue to treat tissue.

## Patentansprüche

1. Chirurgisches System (10), umfassend:
einen Ultraschallwandler (140);
einen Ultraschallwellenleiter (154), der an den Ultraschallwandler gekoppelt ist und sich distal von diesem erstreckt; und
eine Ultraschallklinge (162), die an einem distalen Ende des Ultraschallwellenleiters angeordnet ist und dazu ausgelegt ist, Ultraschallenergie zu empfangen, die von dem Ultraschallwandler erzeugt wird und entlang des Ultraschallwellenleiters übertragen wird, um die Ultraschallklinge zur Behandlung von Gewebe damit zum Schwingen zu bringen, wobei die Ultraschallklinge dazu ausgelegt ist, mit einer Quelle elektrochirurgischer Energie (200) zum Leiten von elektrochirurgischer Energie an Gewebe verbunden zu werden, um Gewebe zu behandeln,
**dadurch gekennzeichnet, dass** die Ultraschallklinge ein Lumen (172) definiert, das sich wenigstens teilweise dadurch bis zu wenigstens einer Öffnung (173) erstreckt, wobei das Lumen für eine Fluidkommunikation mit einer Fluidquelle (170) ausgelegt ist, um die Abgabe von Fluid von der Fluidquelle durch das Lumen und aus der wenigstens einen Öffnung in einen Operationsort zu ermöglichen, um eine elektrochirurgische Gewebebehandlung zu erleichtern.

2. Chirurgisches System gemäß Anspruch 1, ferner umfassend ein Gehäuse (112), wobei der Ultraschallwandler sowie die Fluidquelle an oder in dem Gehäuse angeordnet sind.

3. Chirurgisches System gemäß Anspruch 1, ferner umfassend die Fluidquelle, wobei das Fluid ein elektrisch leitfähiges Fluid ist, das dazu ausgelegt ist, die Ultraschallklinge elektrisch mit Gewebe zu koppeln, wenn die Ultraschallklinge zur elektrochirurgischen Gewebebehandlung verwendet wird.

4. Chirurgisches System gemäß Anspruch 1, wobei das Schwingen der Ultraschallklinge die Ultraschallklinge erhitzt, und wobei die Abgabe von Fluid von der Fluidquelle durch das Lumen und aus der wenigstens einen Öffnung in einen Operationsort die Ultraschallklinge kühlt.

5. Chirurgisches System gemäß Anspruch 1, ferner umfassend ein Backenelement (164), das relativ zu der Ultraschallklinge von einer beabstandeten Position in eine angenäherte Position bewegt werden kann, um Gewebe dazwischen einzuklemmen.

6. Chirurgisches System gemäß Anspruch 5, wobei das Backenelement einen Strukturkörper (182) und eine Backenauskleidung (184) aufweist, die in dem Strukturkörper angeordnet sind, wobei die Backenauskleidung eine Gewebekontaktfläche definiert, die so positioniert ist, dass sie der Ultraschallklinge in der angenäherten Position entgegensteht.

7. Chirurgisches Instrument gemäß Anspruch 6, wobei der Strukturkörper dazu ausgelegt ist, mit der Quelle elektrochirurgischer Energie verbunden zu werden, und wobei der Strukturkörper und die Ultraschallklinge dazu ausgelegt sind, bipolare elektrochirurgische Energie durch dazwischen angeordnetes Gewebe zu leiten, um Gewebe zu behandeln.

8. Chirurgisches System gemäß Anspruch 6, wobei der Strukturkörper wenigstens eine darauf angeordnete elektrisch leitfähige Oberfläche (188) aufweist, die dazu ausgelegt ist, mit der Quelle elektrochirurgischer Energie verbunden zu werden, und wobei die wenigstens eine elektrisch leitfähige Oberfläche und die Ultraschallklinge dazu ausgelegt sind, bipolare elektrochirurgische Energie durch dazwischen angeordnetes Gewebe zu leiten, um Gewebe zu behandeln.

9. Chirurgisches System gemäß Anspruch 1, wobei die Ultraschallklinge dazu ausgelegt ist, monopolare elektrochirurgische Energie an Gewebe zu leiten, um Gewebe zu behandeln.

10. Chirurgisches System (10), umfassend:
einen Ultraschallwandler (140);
einen Ultraschallwellenleiter (154), der an den Ultraschallwandler gekoppelt ist und sich distal von diesem erstreckt;
eine Ultraschallklinge (162), die an einem distalen Ende des Ultraschallwellenleiters angeordnet ist und dazu ausgelegt ist, Ultraschallenergie zu empfangen, die von dem Ultraschallwandler erzeugt wird und entlang des Ultraschallwellenleiters übertragen wird, um die Ultraschallklinge zur Behandlung von Gewebe damit zum Schwingen zu bringen, wobei die Ultraschallklinge dazu ausgelegt ist, mit einer Quelle elektrochirurgischer Energie (200) zum Leiten von elektrochirurgischer Energie an Gewebe verbunden zu werden, um Gewebe zu behandeln; und
ein Backenelement (182), das relativ zu der Ultraschallklinge von einer beabstandeten Position in eine angenäherte Position bewegt werden kann, um Gewebe dazwischen einzuklemmen,
**dadurch gekennzeichnet, dass**
das Backenelement ein Lumen (176) definiert, das für eine Fluidkommunikation mit einer Fluidquelle (170) ausgelegt ist, um die Abgabe von Fluid von der Fluidquelle, durch das Lumen, aus wenigstens einer in dem Backenelement definierten Öffnung (177) und in einen Operationsort zu ermöglichen, um eine elektrochirurgische Gewebebehandlung zu erleichtern.

11. Chirurgisches System gemäß Anspruch 10, ferner umfassend ein Gehäuse (112), wobei der Ultraschallwandler sowie die Fluidquelle an oder in dem Gehäuse angeordnet sind.

12. Chirurgisches System gemäß Anspruch 10, ferner umfassend die Fluidquelle, wobei das Fluid ein elektrisch leitfähiges Fluid ist, das dazu ausgelegt ist, die Ultraschallklinge elektrisch mit Gewebe zu koppeln, wenn die Ultraschallklinge zur elektrochirurgischen Gewebebehandlung verwendet wird.

13. Chirurgisches System gemäß Anspruch 10, wobei das Schwingen der Ultraschallklinge die Ultraschallklinge erhitzt, und wobei die Abgabe von Fluid von der Fluidquelle durch das Lumen und aus der wenigstens einen Öffnung auf die Ultraschallklinge gerichtet wird, um die Ultraschallklinge zu kühlen.

14. Chirurgisches System gemäß Anspruch 10, wobei das Backenelement einen Strukturkörper (182) und eine Backenauskleidung (184) aufweist, die in dem Strukturkörper angeordnet sind, wobei die Backenauskleidung eine Gewebekontaktfläche definiert, die so positioniert ist, dass sie der Ultraschallklinge in der angenäherten Position entgegensteht, wobei optional der Strukturkörper dazu ausgelegt ist, mit der Quelle elektrochirurgischer Energie verbunden zu werden, und wobei der Strukturkörper und die Ultraschallklinge dazu ausgelegt sind, bipolare elektrochirurgische Energie durch dazwischen angeordnetes Gewebe zu leiten, um Gewebe zu behandeln, oder
wobei der Strukturkörper wenigstens eine darauf angeordnete elektrisch leitfähige Oberfläche (188) aufweist, die dazu ausgelegt ist, mit der Quelle elektrochirurgischer Energie verbunden zu werden, und wobei die wenigstens eine elektrisch leitfähige Oberfläche und die Ultraschallklinge dazu ausgelegt sind, bipolare elektrochirurgische Energie durch dazwischen angeordnetes Gewebe zu leiten, um Gewebe zu behandeln.

15. Chirurgisches System gemäß Anspruch 10, wobei die Ultraschallklinge dazu ausgelegt ist, monopolare elektrochirurgische Energie an Gewebe zu leiten, um Gewebe zu behandeln.

## Revendications

1. Système chirurgical (10) comprenant :
un transducteur ultrasonore (140) ;
un guide d'ondes ultrasonores (154) couplé au transducteur ultrasonore et s'étendant de manière distale à partir de celui-ci ; et
une lame ultrasonore (162) disposée à une extrémité distale du guide d'ondes ultrasonores et configurée pour recevoir l'énergie ultrasonore produite par le transducteur ultrasonore et transmise le long du guide d'ondes ultrasonores pour faire vibrer la lame ultrasonore afin de traiter un tissu, la lame ultrasonore étant configurée pour se connecter à une source d'énergie électrochirurgicale (200) afin de conduire l'énergie électrochirurgicale vers un tissu pour traiter le tissu,
**caractérisé en ce que** la lame ultrasonore définit une lumière (172) qui la traverse au moins partiellement jusqu'à au moins une ouverture (173), la lumière étant configurée pour une communication fluidique avec une source de fluide (170) afin de permettre l'administration de fluide à partir de la source de fluide à travers la lumière et par l'au moins une ouverture dans un site chirurgical pour faciliter le traitement électrochirurgical de tissu.

2. Système chirurgical selon la revendication 1, comprenant en outre un boîtier (112), le transducteur ultrasonore et la source de fluide étant disposés sur ou à l'intérieur du boîtier.

3. Système chirurgical selon la revendication 1, comprenant en outre la source de fluide, le fluide étant un fluide électriquement conducteur configuré pour coupler électriquement la lame ultrasonore avec le tissu lorsque la lame ultrasonore est utilisée pour le traitement électrochirurgical de tissu.

4. Système chirurgical selon la revendication 1, la vibration de la lame ultrasonore chauffant la lame ultrasonore, et l'administration de fluide à partir de la source de fluide à travers la lumière et par l'au moins une ouverture dans un site chirurgical refroidissant la lame ultrasonore.

5. Système chirurgical selon la revendication 1, comprenant en outre un élément de mâchoire (164) mobile par rapport à la lame ultrasonore d'une position espacée à une position rapprochée pour serrer un tissu entre celles-ci.

6. Système chirurgical selon la revendication 5, l'élément de mâchoire comprenant un corps structurel (182) et un revêtement de mâchoire (184) disposé dans le corps structurel, le revêtement de mâchoire définissant une surface de contact avec le tissu positionnée pour s'opposer à la lame ultrasonore dans la position rapprochée.

7. Instrument chirurgical selon la revendication 6, le corps structurel étant configuré pour se connecter à la source d'énergie électrochirurgicale et le corps structurel et la lame ultrasonore étant configurés pour conduire l'énergie électrochirurgicale bipolaire à travers un tissu disposé entre les deux pour traiter le tissu.

8. Système chirurgical selon la revendication 6, le corps structurel comprenant au moins une surface électriquement conductrice (188) disposée sur celui-ci, qui est configurée pour se connecter à la source d'énergie électrochirurgicale, et l'au moins une surface électriquement conductrice et une lame ultrasonore étant configurées pour conduire une énergie électrochirurgicale bipolaire à travers un tissu disposé entre les deux pour traiter le tissu.

9. Système chirurgical selon la revendication 1, la lame ultrasonore étant configurée pour conduire l'énergie électrochirurgicale monopolaire vers un tissu pour traiter le tissu.

10. Système chirurgical (10) comprenant :
un transducteur ultrasonore (140) ;
un guide d'ondes ultrasonores (154) couplé au transducteur ultrasonore et s'étendant de manière distale à partir de celui-ci ;
une lame ultrasonore (162) disposée à une extrémité distale du guide d'ondes ultrasonores et configurée pour recevoir l'énergie ultrasonore produite par le transducteur ultrasonore et transmise le long du guide d'ondes ultrasonores pour faire vibrer la lame ultrasonore afin de traiter un tissu, la lame ultrasonore étant configurée pour se connecter à une source d'énergie électrochirurgicale (200) afin de conduire l'énergie électrochirurgicale vers un tissu pour traiter le tissu ; et
un élément de mâchoire (182) mobile par rapport à la lame à ultrasons depuis une position espacée jusqu'à une position rapprochée pour serrer un tissu entre celles-ci,
**caractérisé en ce que**
l'élément de mâchoire définit une lumière (176) configurée pour la communication fluidique avec une source de fluide (170) afin de permettre l'administration de fluide à partir de la source de fluide, à travers la lumière, par au moins une ouverture (177) définie dans l'élément de mâchoire, et dans un site chirurgical pour faciliter le traitement électrochirurgical de tissu.

11. Système chirurgical selon la revendication 10, comprenant en outre un boîtier (112), le transducteur ultrasonore et la source de fluide étant disposés sur ou à l'intérieur du boîtier.

12. Système chirurgical selon la revendication 10, comprenant en outre la source de fluide, le fluide étant un fluide électriquement conducteur configuré pour coupler électriquement la lame ultrasonore avec le tissu lorsque la lame ultrasonore est utilisée pour le traitement électrochirurgical de tissu.

13. Système chirurgical selon la revendication 10, la vibration de la lame ultrasonore chauffant la lame ultrasonore, et l'administration de fluide à partir de la source de fluide à travers la lumière et par l'au moins une ouverture étant dirigé vers la lame ultrasonore pour refroidir la lame ultrasonore.

14. Système chirurgical selon la revendication 10, l'élément de mâchoire comprenant un corps structurel (182) et un revêtement de mâchoire (184) disposé dans le corps structurel, le revêtement de mâchoire définissant un surface de contact avec le tissu positionnée pour s'opposer à la lame ultrasonore dans la position rapprochée, éventuellement le corps structurel étant configuré pour se connecter à la source d'énergie électrochirurgicale, et le corps structurel et la lame ultrasonore étant configurés pour conduire l'énergie électrochirurgicale bipolaire à travers un tissu disposé entre les deux pour traiter le tissu, ou
le corps structurel comprenant au moins une surface conductrice électrique (188) disposée sur celui-ci, qui est configurée pour se connecter à la source d'énergie électrochirurgicale, et au moins une surface électriquement conductrice et une lame ultrasonore étant configurées pour conduire une énergie électrochirurgicale bipolaire à travers un tissu disposé entre les deux pour traiter le tissu.

15. Système chirurgical selon la revendication 10, la lame ultrasonore étant configurée pour conduire l'énergie électrochirurgicale monopolaire vers un tissu pour traiter le tissu.
